# EUROPEAN PATENT APPLICATION

(11) **EP 2 959 870 A1**
(43) Date of publication of application: **30.12.2015**
(21) Application number: 14174826.9
(22) Date of filing: 27.06.2014
(51) Int. Cl.: A61F 5/055, A61F 5/37, A47C 7/38, A61F 5/04, A61F 5/058

(54) **A device for supporting the head of a sitting or standing person in an upright position**

(71) Applicant: Justus, Michael, 20148 Hamburg (DE)
(72) Inventor: Justus, Michael, 20148 Hamburg (DE)
(74) Representative: UEXKÜLL & STOLBERG

(57) **Abstract**

The present invention relates to a device for supporting the head of a sitting or standing person in an upright position. The device comprises a mounting section (2) adapted to be arranged on the upper arm of a user such that the mounting section (2) at least partially surrounds the upper arm and is secured thereto. The device further comprises an at least partially flexible support section (3) comprising two opposite ends. At or near each of the two ends the support section (3) is secured or can be secured to the mounting section (2) in such a manner that the support section (3) forms a loop and that, when the mounting section (2) is secured to the upper arm of a user, the loop extends from an edge of the mounting section (2) facing towards the respective shoulder of the user and the loop can be guided from the mounting section (2) around the head of the user abutting the head at least at a portion opposite from the respective shoulder of the user.

## Description

The present invention relates to a device for supporting the head of a sitting or standing person in an upright position.

It is generally difficult for a person to sleep in a sitting or standing position, because the head falls forwardly or sideways in an uncontrolled manner as soon as the person has fallen asleep. For this reason, without additional means or devices a relaxing sleep is not possible while being in a sitting position or also while standing up. This is a considerable problem, for example for travelers in aircraft, cars and trains.

Attempts have been made to solve these problems by means of various different supporting devices, such as particularly shaped cushions or frames. However, these solutions have turned out not to be satisfactory, because they provide an insufficient or unreliable supporting effect, are of complex construction, are difficult to use and/or have significant space requirements making it difficult for a traveler to take them on a trip. Furthermore, they often cause an inconvenient and sometimes painful pressure on sensitive areas of the body, such as, for example, the temple, the jaw, the back of the neck, the vertebrae, etc., which pressure may keep the user from an extended period of rest and, upon prolonged period of use, may even result in negative health effects.

It is an object of the present invention to provide a device for supporting the head of a sitting or even standing person in an upright position, which device is of simple construction, can be made to be of low weight and is easy to use and to transport, and which nevertheless provides a suitable and reliable support effect.

This object is achieved by a device for supporting the head of a sitting or standing person in an upright position as defined in claim 1. Preferred embodiments of the device are the subject-matter of the dependent claims.

According to the present invention, a device for supporting the head of a sitting or standing person in an upright position comprises or consists of a mounting section or portion and an at least partially flexible support section or portion, which is preferably elongate.

The mounting section is adapted to be arranged on the upper arm of a user such that the mounting section at least partially surrounds the upper arm, i.e. extends in the circumferential direction of the upper arm and partially or completely encloses the upper arm in the circumferential direction, and is thereby securely mounted to the upper arm. In particular, the mounting section may take the form of a sleeve, cuff or bangle.

The support section comprising two opposite longitudinal ends. At or near each of these two ends the support section is secured or connected or can be secured or connected to the mounting section in such a manner that the support section forms a loop and that, when the mounting section is secured to the upper arm of a user in the manner described above, the loop extends from an edge of the mounting section facing towards the shoulder of the user corresponding to the upper arm to which the mounting section is applied and the loop can be guided from the mounting section around the head of the user abutting the head at least at a portion opposite from the respective shoulder of the user. Thus, in the applied state of the device the loop extends from the same edge for both ends or end regions of the support section, and the support section extends from the mounting section, towards the head of the user, around a portion of the head of the user and back to the mounting section. Consequently, the head is supported by the support section against movement away from the upper arm to which the mounting section is secured, so that the user can "lean into" the loop and thereby support her or his head in an upright position even after falling asleep. In view of the above, the support section is a loop section or portion or can be brought into a configuration in which it is a loop portion or section, which loop portion or section is adapted to be used in the described manner.

This device, which is of a very simple construction and can be made to have low weight can be applied by a sitting or standing person in a simple manner and without assistance by at first mounting the mounting section to one upper arm and then suitably arranging the support section in a loop around the head. It is also very simple to take off the device after use. Furthermore, the device only has very little space requirements. Also, by adjusting the rotational position of the mounting section, i.e. by rotating the mounting section in the circumferential direction thereof and of the upper arm, it is possible to adapt the orientation and position of the loop, so that it is easily possible to guide the loop around the head in different configurations and around different portions of the head. For example, in a first possible configuration the entire loop extends behind the neck and only around the back of the head, in a second possible configuration the loop extends from the mounting section over the cheek opposite the upper arm to which the mounting section is secured and around the head back to the mounting section, and in a third possible configuration the loop extends from the mounting section over the face and around the head back to the mounting section. In addition to these three possibilities, which have been found to be particularly effective, there exist other possible configurations during use. Further, the device may be easily configured to provide for additional functionality, e.g. by providing a pocket for a portable music player or mobile phone in the mounting section or the support section.

In a preferred embodiment the mounting section is an annularly closed ring, structure or arrangement, which - without destroying it - cannot be opened or separated into a configuration in which it is not annularly closed. In an alternative preferred embodiment the mounting section is constituted by a - preferably partially or entirely flexible and preferably flat or sheet-like - structure or arrangement which can be selectively brought into an annularly closed configuration by securing different portions and preferably opposite end portions thereof to each other. In particular, the mounting section may comprise an elongate element which can be interconnected at end sections or in end regions thereof to form an annularly closed ring, structure or arrangement. For this purpose, suitable fastening means may be provided, such as Velcro fastening means, hook-and-loop fastening means or buttons. Embodiments in which an annularly closed ring, structure or arrangement, e.g. a sleeve, can be opened are advantageous, because it facilitates putting on the mounting section and the entire device. In other words, according to the two embodiments the mounting section is a sleeve section or portion or can be brought into a configuration in which it is a sleeve portion or section, which sleeve portion or section is adapted to be worn on and secured to the upper arm of a person.

In both of the two preceding embodiments, it is preferred if the mounting section is constructed such that a diameter of the annularly closed ring, structure or arrangement is adjustable. For example, in the case of the above embodiment, in which the mounting section comprises a structure or arrangement which can be selectively brought into an annularly closed configuration, e.g. an elongate element which can be interconnected at end sections thereof to form the annularly closed ring, end sections or regions thereof may comprise fastening elements allowing the end sections or regions to be secured to each other in various degrees of overlap. Such fastening elements may, e.g., be cooperating Velcro fastening elements or cooperating hook and loop fastening elements. However, it is also possible for the mounting section to be adjustable in circumferential length by providing an adjustment buckle or an adjustment loop. Adjustability of the diameter of an annularly closed ring, structure or arrangement formed by the mounting section facilitates putting on the mounting section and securely mounting the mounting section to persons having different upper arm diameters. In a further preferred version of the above embodiments the mounting section is made of or comprises an elastic material, such as for example Neopren, allowing for elastically expanding the diameter of the annularly closed ring. This also allows adapting the mounting section to different persons and facilitates fixedly securing the mounting section to an upper arm.

Independent of whether the mounting section is annularly closed or can be arranged in an annularly closed state, in a preferred embodiment the mounting section comprises or is made of a flexible material, thereby providing the advantages of easy of application, adaptability and enhancing the mounting security. It should be noted that the mounting section may or may not also comprise a rigid or semi-rigid portion. Overall, even if the mounting section is made of or comprises flexible material, it is preferred if the mounting section has some degree of rigidity in order to contribute in preventing the mounting section from cutting into or constricting the upper arm during use. For the same purpose, it is additionally or alternatively preferred if the mounting section has a certain width. The latter aspect also applies to all embodiments.

In an alternative embodiment, the mounting section may comprise or be made of a semi-rigid material, i.e. a material being rigid but being plastically deformable or preferably having some degree of elasticity, or a rigid material. In this embodiment it is particularly preferred if the mounting section has the shape of an open ring, i.e. a section of a ring-shaped structure, or if the mounting section comprises a portion or element made of the semi-rigid or rigid material and having the shape of an open ring. In these cases it is particularly preferred if the mounting section or the portion or element, as the case may be, is constructed such that in the applied state it extends over more than 50% of the circumference of the upper arm, thereby being capable of retaining itself on the upper arm without additional means. In the case of a rigid material having elastic properties the mounting section may therefore be adapted to be "clipped onto" an upper arm of a user.

Thus, the mounting section may be flexible, semi-rigid or rigid in its entirety, or it may comprise multiple portions, elements or components, each of which may be flexible, semi-rigid or rigid, and each of which may be of the same or different materials. Thus, one or more flexible portions, elements or components may be combined with one or more semi-rigid or rigid portions, elements or components.

Generally, in preferred embodiments the mounting section may consist of a single element or component only, and in other preferred embodiments it may comprise two or more elements or components. For example, the mounting section may have a multi-layered structure. All of such portions, elements or components may be fixedly secured to each other or detachable from each other. For example, a cushioning element or portion adapted to provide comfort to the user may be combined with other elements or portions. Further, each such portion, element or component may be configured such that at least one dimension thereof is adjustable, thereby providing the mounting section as a whole with adjustability, in particular for allowing adapting the mounting section and the entire device to a particular user. The above also comprises that the entire mounting section or at least a portion, element or component thereof has elastic properties, thereby allowing adjustability. However, it is also possible that the mounting section is not adjustable or adaptable.

In a preferred embodiment the mounting section comprises or is made of Neoprene.

The width of the mounting section, i.e. the dimension of the mounting section along the extension of the upper arm in the applied state, may preferably be 8 to 10 cm and even more preferably about 9 cm. The length of the mounting section in the circumferential direction is chosen such that it can be secured in the manner described above to a human upper arm. In case of embodiments in which the mounting section can be opened from an annularly closed state, e.g. the above-described embodiments in which the mounting section is constituted by a - preferably partially or entirely flexible and preferably flat or sheet-like - structure or arrangement such as an elongate element, which can be selectively brought into an annularly closed configuration by securing different portions and preferably opposite end portions thereof to each other, the length of the structure of arrangement in the direction defining the circumferential direction in use may preferably be 45 to 50 cm. These dimensions allow for an easy adaptability of the mounting section to human upper arms. In case the mounting section cannot be opened from an annularly closed state, the circumference of the corresponding annularly closed ring, structure or arrangement may be, e.g., 20 to 40 cm. This also applies to a mounting section, which can be opened, when mounted to an upper arm of a user.

It may also be advantageous if the mounting section is inflatable or comprises an inflatable portion, component or element, thereby providing adaptability or additional adaptability to different users and potentially enhancing the comfort for the user.

In a preferred embodiment the support section is strip- or strap-shaped or at least comprises a strip- or strap-shaped portion. Such a support section or strip- or strap-shaped portion provides an extended abutment surface for abutting the head of the user. During use, the abutment surface provides an extended contact or support surface which contacts the user and supports the device against the user. Due to the extension of the abutment surface it can be provided that the contact between the head and the support section is hardly noticeable for the user and that no inconvenient or harmful pressure is exerted on sensitive regions of the body. It is to be noted that it is also possible to configure the support section as a chord or chain provided with a cushioning element in the region of the head of the user.

In a preferred embodiment the length of the loop is adjustable. For this purpose, the length of the support section itself may be adjustable, e.g. by means of Velcro means or adjustment buckles. Additionally or alternatively it is possible that one or both ends of the support section can be releasably secured or connected to the mounting section at different positions, or that the mounting section is connected at different positions along the length of the support section near the longitudinal ends thereof.

Generally, the support section may be flexible in its entirety, or it may be partially flexible by comprising multiple portions, elements or components, each of which may be flexible, semi-rigid or rigid, and each of which may be of the same or different materials. Thus, one or more flexible portions, elements or components may be combined with one or more semi-rigid or rigid portions, elements or components to render the support section partially flexible.

Generally, in preferred embodiments the support section may consist of a single element or component only, and in other preferred embodiments it may comprise two or more elements or components. For example, the support section may have a multilayered structure. All of such portions, elements or components may be fixedly secured to each other or detachable from each other. For example, a cushioning element or portion adapted to provide comfort to the user may be combined with other elements or portions. Further, each such portion, element or component may be configured such that at least one dimension thereof is adjustable, thereby providing the support section as a whole with adjustability, in particular for allowing adapting the support section and the entire device to a particular user. The above also comprises that the entire support section or at least a portion, element or component thereof has elastic properties, thereby allowing adjustability. However, it is also possible that the support section is not adjustable or adaptable.

In a preferred embodiment the mounting section comprises or is made of Nylon.

The width of the support section, i.e. the dimension of the support section perpendicular to the direction along the loop or - in the applied state during use - to the direction from the mounting section towards the head of the user and back to the mounting section, may preferably be 4 to 6 cm and even more preferably about 5 cm. The maximum length of the support section in the direction of the loop or the maximum length of the support section from the mounting section, along the loop and back to the mounting section, and in particular the maximum adjustable length, may preferably be 70 to 90 cm and even more preferably about 80 cm.

In a preferred embodiment, at or near each of the two ends of the support section the support section is or can be secured or connected to the mounting section at the same location, at immediately adjacent locations, or at spaced locations in the circumferential direction of the mounting section. It is preferred if in use the support section is secured or connected to the mounting section at opposite locations or essentially opposite locations with respect to the upper arm of the user.

In a preferred embodiment, at or near one or both of the two ends of the support section the support section, or at least a part thereof, is integrally formed in one piece with the mounting section. Although this may apply to both ends, it is preferred that at or near one of the two ends the support section can be released from the mounting section in order to facilitate putting on the device.

In an alternative preferred embodiment the mounting section and the support section are separate elements, and at or near one or both of the two ends of the support section the support section is fixedly secured to the mounting section, i.e. such that it is not releasable. Again, although this may apply to both ends, it is preferred that at or near one of the two ends the support section can be released from the mounting section in order to facilitate putting on the device.

In a preferred embodiment, at or near one or both of the two ends of the support section the support section is or can be releasably secured or connected to the mounting section. As noted above, this facilitates putting on the device. For the purpose of releasably securing or connecting the support section to the mounting section, the support section and the mounting section are preferably provided with mating fastening elements, such as, in particular, Velcro fastening elements, hook and loop fastening elements, snap fasteners, buttons, clamping elements, adjustment buckles, etc.

In a preferred embodiment the support section is or can be secured or connected to the mounting section at or near one or both of the ends of the support section via an intermediate element, for example a ring-shaped element. In particular, such intermediate elements may comprise or be made of an elastic material. Due to the elastic material they enhance the comfort for the user and allow for dampening of vibrations, such as vibrations present in an aircraft. When using intermediate elements, it is particularly preferred to provide for an adjustability of the length of the loop by means of an adjustability of the length of the support section itself, e.g. by means of adjustment buckles, as explained above.

It may generally be advantageous if the mounting section and/or the support section comprises an integrated or releasably connected cushioning element, which is arranged or can be arranged such that in use the cushioning element abuts the upper arm and the head, respectively.

In the following, an exemplary embodiment of the device is described in more detail with reference to the drawings.
Figure 1 shows a perspective view of a device according to an embodiment of the invention.
Figure 2 shows a second perspective view of the device shown in Figure 1.
Figure 3 shows a third perspective view of the device shown in Figure 1.
Figures 4a and 4b show a top plan view and a bottom plan view, respectively, of the device of Figures 1 to 3 with the mounting section being in an open state.
Figures 5a to 5d show different embodiments of mounting sections which can be used with a device according to the present invention.
Figure 6 shows the device of Figures 1 to 4 in a folded condition.
Figures 7a to 7c illustrates three possible configurations in which the device of Figures 1 to 4 may be worn by a user.

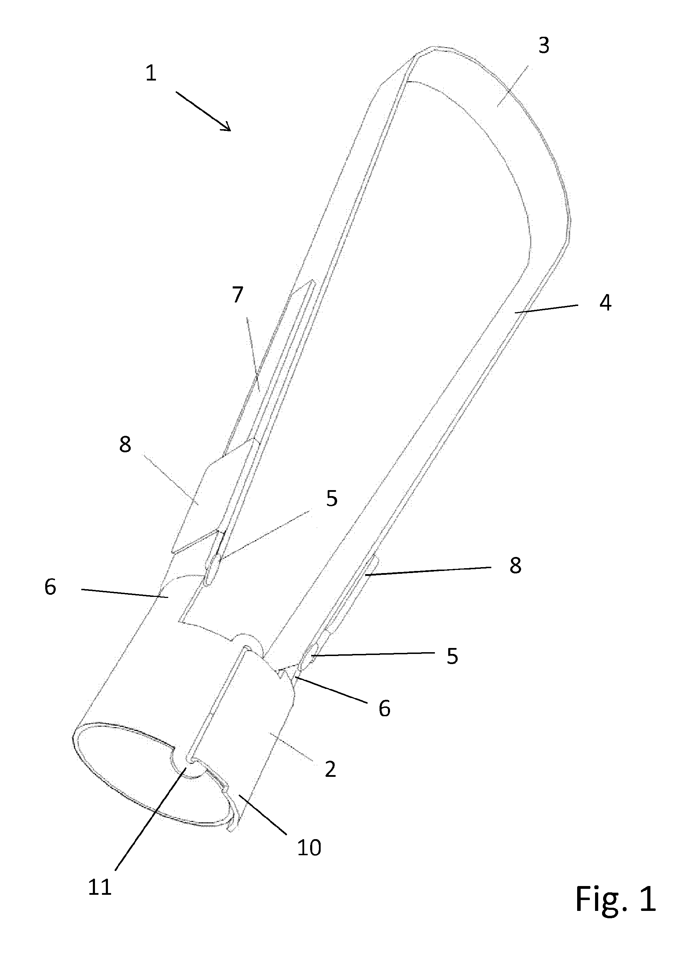

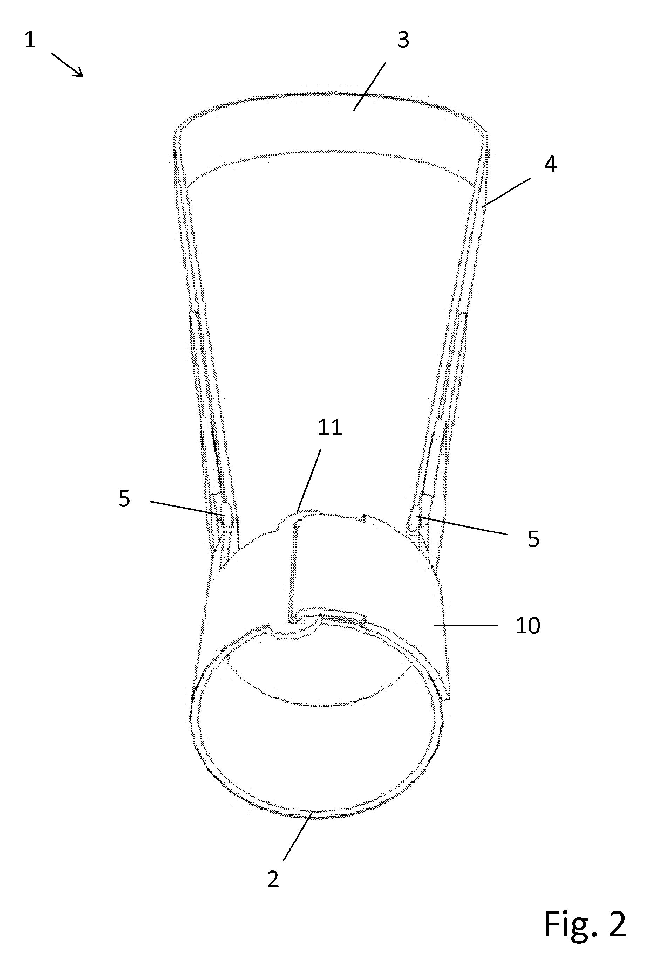

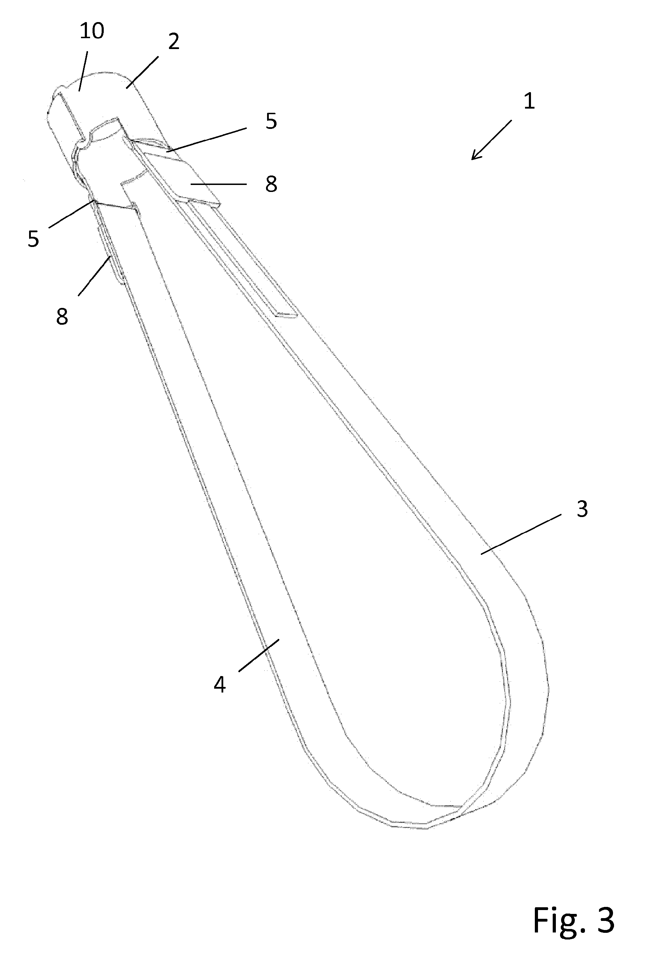

Figures 1 to 3 show different perspective views of a device 1 for supporting the head of a sitting or standing person in an upright position. The device 1 comprises a mounting section 2 and a support section 3. In the configuration shown in Figures 1 to 3 the mounting section 2 constitutes a sleeve portion and the support section 3 constitutes a loop portion, which loop portion is connected to the sleeve portion such that the sleeve portion closes the loop. The mounting section 2 and the support section 3 are separate elements which may be configured to be detachable from each other, if desired.

The support section 3 comprises an elongate strip 4, which is preferably made of Nylon material and in particular Nylon webbing, and is releasably connected in each of its two opposite longitudinal end regions to the mounting section via an intermediate component 5 made of or comprising an elastic material and being in turn coupled to both the mounting section 2 and a respective end region of the strip 4. In the illustrated embodiment these intermediate components 5 are provided in the form of buckles, but may take any form having an annularly closed portion through which the end regions of the strip 4 can be guided in the manner described in the following.

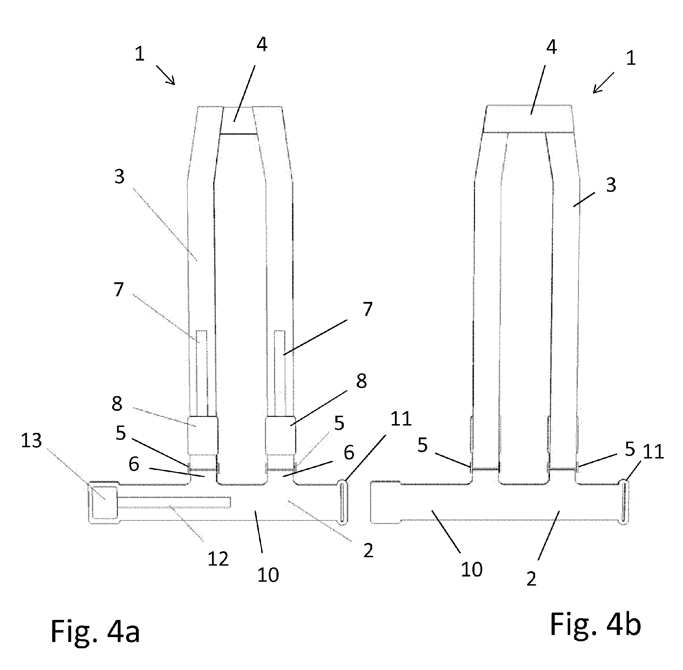

As can be seen best in Figures 1 and 4, each of the buckles 5 is fixedly secured to a corresponding projection 6 of the mounting section 2. Further, each of the opposite end regions of the strip 4 is guided through the associated buckle 5, looped back onto itself, and the portions of the end region before and after the buckle 5 are attached to each other by means of a respective Velcro strip 7 attached to the strip 4 in each of the two end regions thereof. Therefore, the effective length of the strip 4, and thus the dimensions of the loop formed by the support section 3, can be easily adjusted by changing the length of the portion of the end region (s) looped back onto the end region (s). In order to facilitate gripping and handling the strip 4 during this adjustment process, each of the two outer terminal ends of the strip 4 is provided with a gripping pad 8, which may be made, e.g., of rubber material and which comprises a Velcro element (not illustrated) cooperating with the respective Velcro strip 7.

The mounting section 2 likewise comprises an elongate strip 10, which is preferably made of Neoprene material. Its width in a direction perpendicular to the longitudinal direction of the strip 10 is larger than the corresponding width of the strip 4. In order to form a sleeve, the two opposite end regions of the strip 10 are releasably interconnected. For this purpose, a ring-shaped fastening element 11 is integrally formed on one of the two opposite longitudinal ends of the strip 10, and the opposite end region of the strip 10 is guided through the fastening element 11, looped back onto itself, and the portions of the latter end region before and after the fastening element 11 are attached to each other by means of a respective Velcro strip 12 and a cooperating Velcro element 13 attached to the strip 10 in the latter end region. Therefore, the effective length of the strip 10, and thus the diameter of the sleeve formed by the mounting section 2, can be easily adjusted by changing the length of the portion of the end region looped back onto the strip 10. The two projections 6 extends from the same edge of the sleeve at locations spaced from each other in the circumferential direction of the sleeve.

While Figures 1 to 3 show the mounting section 2 in a configuration in which it forms an annularly closed sleeve, Figure 4 shows the mounting section 2 in an open configuration in which the strip 10 may be can be disposed flat on a surface. Starting from the latter configuration the device 1 may be applied to or put on by a user by guiding the strip 10 around one of his or her upper arms, closing the strip 10 into the annularly closed sleeve configuration shown in Figures 1 to 3, and adjusting the diameter of the sleeve such that the device is firmly secured to the upper arm. Of course, it is also possible to start with the device 1 being in the sleeve configuration of Figures 1 to 3, to guide the arm through the sleeve until the mounting section 2 is disposed at a suitable position on the upper arm, and to then tighten the sleeve by adjusting its diameter in the manner described above.

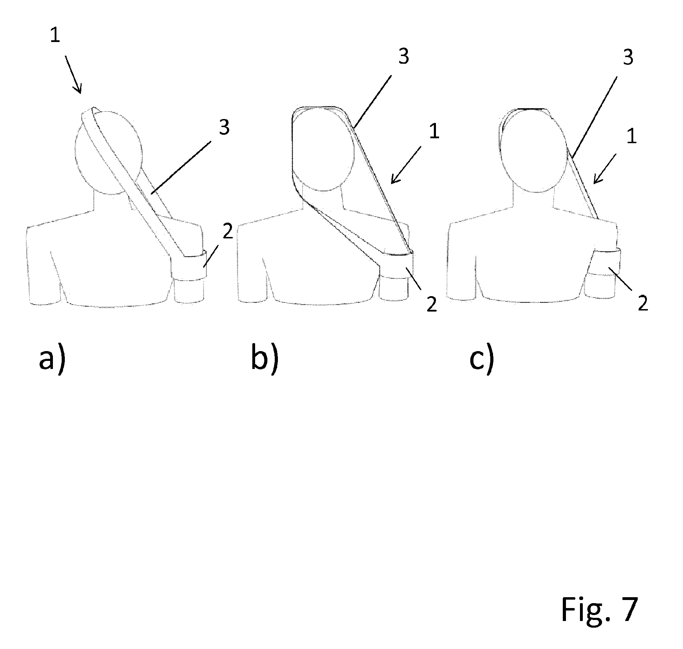

The mounting section 2 is arranged on the upper arm such that the projections 6 are oriented towards the respective shoulder. Thus, as illustrated in Figure 7, the loop provided by the support section 3 may then be guided towards the head of the user, and the head may be disposed inside the loop in such a manner that the strip 4 abuts the head at least in a region opposite the shoulder and the upper arm to which the mounting section is secured. Consequently, the head is supported in an upright position by the device 1 when the user leans with his or her head into the loop.

Figure 7 shows three different possibilities for guiding the loop around the head, which possibilities have been found to be particularly effective and convenient. In Figure 7a the strip 4 extends from the mounting section 2 at opposite sides of the upper arm and extends around the head over the face and over the back of the head. In Figure 7b the strip 4 extends from the mounting section 2 at the front of the upper arm and extends in front of the user around the head over the remote cheek region. In Figure 7c the strip 4 extends from the mounting section 2 at the back of the upper arm and extends entirely behind the back of the user around the back of the head. Other configurations are also possible.

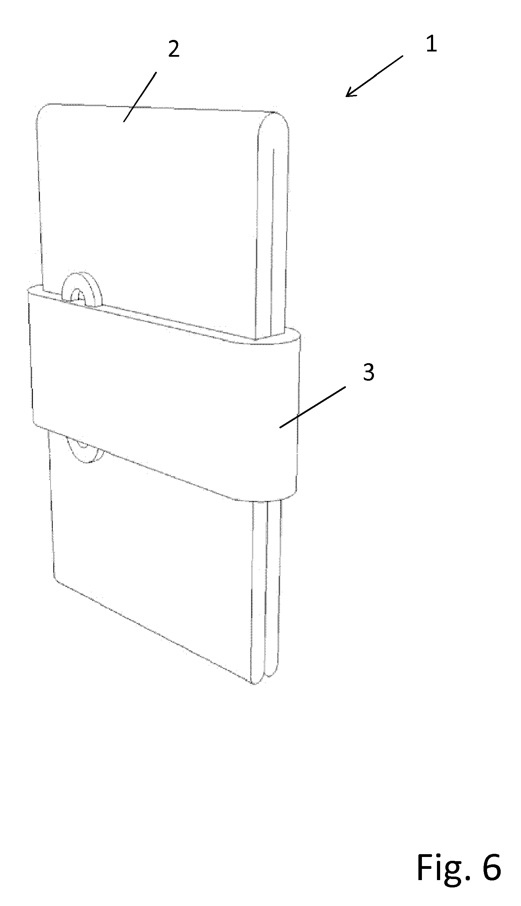

As illustrated in Figure 6, when not in use, the device 1 shown in Figures 1 to 4 can be easily folded into a relatively small and relatively flat bundle, which makes it easy to take it on a trip.

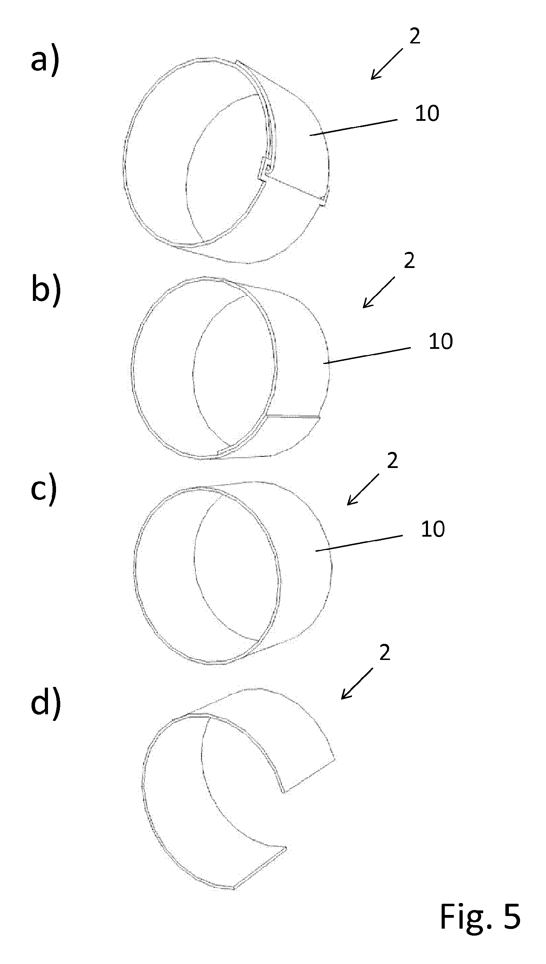

Figure 5 shows four different embodiments of the mounting section 2 (for ease of illustration shown in isolation without the support section 3 and without the projections 6). Figure 5a once again shows the embodiment of the mounting section 2 used in Figures 1 to 4. Figures 5b and 5c show embodiments of the mounting section 2 in which the diameter of the mounting section 2 is not adjustable. In Figure 5b the mounting section 2 again comprises a strip 10, but the opposite end regions thereof are fixedly and non-detachably secured to each other, for example by using a suitable adhesive, and in Figure 5c the mounting section 2 is integrally formed in one piece as an annularly closed structure. In the embodiments of Figures 5b and 5c it is particularly preferred if the sleeve has elastic properties, to thereby guarantee some degree of adaptability to different users and to effect a reliable securing of the mounting section 2 to an upper arm. Finally, in Figure 5d the mounting section 2 is made of a rigid or semi-rigid material and provided in the shape of an open ring.

## Claims

1. A device for supporting the head of a sitting or standing person in an upright position, comprising
- a mounting section (2) adapted to be arranged on the upper arm of a user such that the mounting section (2) at least partially surrounds the upper arm and is secured thereto, and
- an at least partially flexible support section (3) comprising two opposite ends,
wherein at or near each of the two ends the support section (3) is secured or can be secured to the mounting section (2) in such a manner that the support section (3) forms a loop and that, when the mounting section (2) is secured to the upper arm of a user, the loop extends from an edge of the mounting section (2) facing towards the respective shoulder of the user and the loop can be guided from the mounting section (2) around the head of the user abutting the head at least at a portion opposite from the respective shoulder of the user.

2. The device according to claim 1, wherein the mounting section (2) is an annularly closed ring, or wherein the mounting section (2) comprises an elongate element which can be connected at end sections thereof to form an annularly closed ring.

3. The device according to claim 2, wherein the mounting section (2) is arranged such that a diameter of the annularly closed ring is adjustable.

4. The device according to any of the preceding claims, wherein the mounting section (2) comprises a flexible material.

5. The device according to any of the preceding claims, wherein the mounting section (2) comprises a rigid or semi-rigid material and has the shape of an open ring section.

6. The device according to any of the preceding claims, wherein the mounting section (2) is inflatable or comprises an inflatable portion.

7. The device according to any of the preceding claims, wherein the support section (3) is strip- or strap-shaped.

8. The device according to any of the preceding claims, wherein the length of the loop is adjustable.

9. The device according to any of the preceding claims, wherein at or near each of the two ends of the support section (3) the support section (3) is or can be secured to the support section (3) at the same location or at spaced locations in the circumferential direction of the mounting section (2).

10. The device according to any of the preceding claims, wherein at or near at least one of the two ends of the support section (3) the support section (3) is integrally formed in one piece with the mounting section (2).

11. The device according to any of claims 1 to 9, wherein the mounting section (2) and the support section (3) are separate elements, and at or near at least one of the two ends of the support section (3) the support section (3) is fixedly secured to the mounting section (2).

12. The device according to any of the preceding claims, wherein at or near at least one of the two ends of the support section (3) the support section (3) is or can be releasably secured to the mounting section (2).

13. The device according to any of the preceding claims, wherein at or near at least one of the two ends of the support section (3) the support section (3) is or can be secured to the mounting section (2) via an intermediate ring-shaped fastening element (5) which comprises or is made of an elastic material.

14. The device according to any of the preceding claims, wherein the mounting section (2) and/or the support section (3) comprises an integrated or releasable cushioning element.
